# EUROPEAN PATENT APPLICATION

(11) **EP 4 442 287 A1**
(43) Date of publication of application: **09.10.2024**
(21) Application number: 22901259.6
(22) Date of filing: 29.11.2022
(51) Int. Cl.: A61L 27/18, A61L 29/06, A61L 31/06, C08G 18/28, C08G 18/44, C08G 18/48, C08G 18/73, C08G 18/75, C08G 18/76

(54) **MEDICAL DEVICE**

(30) Priority: 01.12.2021 JP 2021195737
(71) Applicant: DKS Co. Ltd., Kyoto-shi, Kyoto 600-8873 (JP); Kyushu University, National University Corporation, Nishi-ku Fukuoka-shi Fukuoka 819-0395 (JP)
(72) Inventor: NISHIMURA Takuma, kyoto-shi, Kyoto 600-8873 (JP); SHIOJI Yudai, kyoto-shi, Kyoto 600-8873 (JP); TANABE Shichidai, kyoto-shi, Kyoto 600-8873 (JP); NISHIURA Masahito, Kyoto-shi, Kyoto 600-8873 (JP); KOBAYASHI Shingo, Fukuoka-shi, Fukuoka 819-0395 (JP); TANAKA Masaru, Fukuoka-shi, Fukuoka 819-0395 (JP)
(74) Representative: Hoffmann Eitle
(86) International application number: PCT/JP2022/043843
(87) International publication number: WO 2023/100827

(57) **Abstract**

Provided is a medical device containing a polyurethane resin elastomer that can achieve both biocompatibility and physical properties. The medical device in an embodiment contains a polyurethane resin elastomer and water contained in the polyurethane resin elastomer. The polyurethane resin elastomer contains polyethylene glycol and a polyisocyanate as constituents, the polyethylene glycol has a number average molecular weight of 200 or more and 2200 or less, and the polyethylene glycol is present in an amount of 1 mass% or higher and lower than 50 mass% based on 100 mass% of the constituents of the polyurethane resin elastomer.

## Description

### Technical Field

The present invention relates to a medical device, and more specifically, to a device used in contact with biological tissue or body fluid.

### Background Art

Generally, it is known that biological tissue or blood that has come into contact with the surface of a substance that does not originate from a living organism recognizes the surface as a foreign substance, and that proteins in the biological tissue non-specifically adsorb onto the surface and denature, while activation of the coagulation system, complement system, platelet system, etc. occurs, leading to adsorption of blood cells, such as platelets, on the surface.

Recent years have seen attention drawn to the state of water molecules present on the surface of synthetic polymers of a specific structure. It is being clarified that forming a surface that can contain water molecules that moderately interact with a polymer material on the surface of the polymer material makes it possible to prevent non-specific adsorption of proteins onto the surface and denaturation of the adsorbed proteins. It is also becoming clear that such a surface has a low adhesion frequency of platelets upon contact with fluid such as blood and can inhibit inflammation that occurs upon contact with biological tissue (see, for example, NPL 1).

The water molecules that moderately interact with a polymer material present as described above inhibit nonspecific adsorption of proteins on the surface of the substance, thereby mitigating various problems that may occur in biological tissue or blood that comes into contact with the material. This phenomenon is generally described as being "biocompatible" (hemocompatible).

PTL 1 discloses a biocompatible hydrated polymer gel derived from prepolymer units, at least 75% of which are oxyethylene-based polyols having a molecular weight of 7000 to 30000, wherein the hydroxyl groups of the polyols are capped with a polyisocyanate.

### Citation List

### Patent Literature

PTL 1: JPH01-271410A

### Non-patent Literature

NPL 1: Journal of The Adhesion Society of Japan, Vol. 51, No. 9, (2015), pp. 15 to 25

### Summary of Invention

### Technical Problem

Polymers containing polyethylene glycol as a constituent are biocompatible. Also, equipment used in contact with biological tissue may be required to have physical properties such as tensile strength along with biocompatibility such as hemocompatibility. However, while a polyurethane resin elastomer containing a large amount of polyethylene glycol as a constituent can be biocompatible, its physical properties such as tensile strength could be impaired.

Given the circumstances above, an object of the embodiments of the present invention is to provide a medical device containing a polyurethane resin elastomer that can achieve both biocompatibility such as hemocompatibility and physical properties.

### Solution to Problem

The present invention includes the following embodiments.
[1] A medical device comprising
   a polyurethane resin elastomer, and
   water contained in the polyurethane resin elastomer,
      the polyurethane resin elastomer comprising
         polyethylene glycol and a polyisocyanate as constituents,
         wherein
      the polyethylene glycol has a number average molecular weight of 200 or more and 2200 or less, and
      the polyethylene glycol is present in an amount of 1 mass% or higher and lower than 50 mass% based on 100 mass% of the constituents of the polyurethane resin elastomer.
[2] The medical device according to [1], wherein the content of the water is higher than 1 mass% and lower than 50 mass% based on the polyurethane resin elastomer.
[3] The medical device according to [1] or [2], wherein the polyisocyanate contains at least one member selected from the group consisting of hexamethylene diisocyanate, diphenylmethane diisocyanate, methylenebis(4,1-cyclohexylene)=diisocyanate, isophorone diisocyanate, and modified forms thereof.
[4] The medical device according to any one of [1] to [3], wherein the polyurethane resin elastomer further comprises a polycarbonate diol as a constituent.
[5] The medical device according to any one of [1] to [4], wherein the polyurethane resin elastomer is a coating film or molded article with a thickness of more than 1 mm.
[6] The medical device according to any one of [1] to [5], wherein the polyurethane resin elastomer is formed without becoming an emulsion.
[7] The medical device according to any one of [1] to [6], wherein the polyurethane resin elastomer is obtained by performing molding while reacting a polyol with the polyisocyanate.
[8] The medical device according to any one of [1] to [7], wherein the polyurethane resin elastomer is thermoplastic.
[9] The medical device according to any one of [1] to [8], wherein the polyurethane resin elastomer further comprises a monohydric alcohol containing a fluorine atom as a constituent.

### Advantageous Effects of Invention

The embodiments of the present invention provide a medical device containing a polyurethane resin elastomer that can achieve both biocompatibility and physical properties.

### Description of Embodiments

The medical device in the present embodiment contains a polyurethane resin elastomer and water contained in the polyurethane resin elastomer, wherein the polyurethane resin elastomer contains polyethylene glycol and a polyisocyanate as constituents.

The medical device in the present embodiment is a device, instrument, or machine for applications in which the medical device is used in contact with biological tissue or body fluid (e.g., blood, lymph, or tissue fluid), including various medical devices required to be biocompatible.

Specific examples of medical devices include blood filters, dialysis machines, blood storage bags, platelet storage bags, blood circuits, artificial bones, artificial blood vessels, artificial organs (e.g., artificial lungs, artificial hearts, artificial pancreases, and artificial livers, which may be implanted in the body or an extracorporeal circulatory system), pacemakers, indwelling needles, catheters (e.g., cardiovascular catheters such as angiographic catheters and PTCA catheters, digestive catheters such as gastric tube catheters, gastrointestinal catheters, and esophageal catheters, tubes, urological catheters, such as urethral catheters and urinary tube catheters), guidewires, stents, endoscopes, repair materials for tissue regeneration, carriers for sustained drug release systems, embolization materials, and matrix materials for scaffolds for cell engineering. The medical device may also be a medical instrument or machine that includes these as components. These medical devices may be formed of the water-containing polyurethane resin elastomer in their entirety or may be formed of the water-containing polyurethane resin elastomer in at least a portion of the area that comes in contact with biological tissue or body fluid, preferably in the entirety of the contact area.

In the present embodiment, a polyurethane resin elastomer containing polyethylene glycol as a constituent manifests a high level of biocompatibility. In the present specification, the term "biocompatibility" refers to properties of a substance being less likely to be recognized as a foreign substance upon contact with a biological substance or a substance of biological origin, such as inhibited adhesion frequency of platelets or inhibited non-specific adsorption of proteins. Specifically, the term means, for example, not causing complement activation or platelet activation and being minimally invasive or noninvasive to tissue. The term "biocompatibility" also includes the embodiment of "hemocompatibility"; the term "hemocompatibility" mainly means not causing blood coagulation due to adhesion or activation of platelets.

The mechanism by which the polyurethane resin elastomer in the present embodiment manifests biocompatibility is unknown. The structural measurement of water contained in the polyurethane resin elastomer according to the present embodiment by using a differential scanning calorimeter (DSC) found the presence of water that melts below 0°C during a temperature increase process (the temperature increase rate: about 5°C/min), suggesting the presence of water molecules that interact moderately with the polyurethane resin elastomer. In the present specification, water that melts at temperatures below 0°C is referred to as "intermediate water (low-temperature-melting water)." The presence of intermediate water is thought to bring about biocompatibility (hemocompatibility), such as the inhibition of platelet adsorption.

The polyurethane resin elastomer in the present embodiment contains polyethylene glycol (PEG) and a polyisocyanate as constituents. The "constituent" of the polyurethane resin elastomer refers to a starting material of the polyurethane resin elastomer incorporated as part of the polymer chain constituting the polyurethane resin elastomer. Thus, the constituent is not polyethylene glycol or a polyisocyanate itself at the stage of incorporation into the polymer chain, but has a structure derived from them (e.g., a polyoxyethylene structure for polyethylene glycol).

The polyethylene glycol for the present embodiment has a number average molecular weight (Mn) of 200 or more and 2200 or less. A polyethylene glycol with a number average molecular weight of 2200 or less can limit the decrease in strength of the polyurethane resin elastomer. The polyethylene glycol preferably has a number average molecular weight of 300 or more, more preferably 400 or more, or 500 or more. The polyethylene glycol preferably has a number average molecular weight of 2000 or less, more preferably 1500 or less, and still more preferably 1000 or less, or 800 or less.

In the present specification, the number average molecular weight is measured by the GPC method (gel permeation chromatography) and is a value calculated from the elution time of the measured sample using a calibration curve created from the molecular weight and elution time of standard polystyrene. In the Examples below, measurement was performed under the conditions of column: TSKgel HXL (Tosoh Corporation); mobile phase: THF; mobile phase flow rate: 1.0 mL/min; column temperature: 40°C; sample injection volume: 50 µL; and sample concentration: 0.2 mass%.

The polyethylene glycol for use may be linear polyethylene glycol (linear PEG), branched polyethylene glycol (branched PEG), or a combination of linear PEG and branched PEG. As used herein, "linear PEG" refers to a polyol with a hydroxyl group at each end of a linear polyoxyethylene structure. Using linear PEG can introduce a polyoxyethylene structure into the main polymer chain of the polyurethane resin elastomer because of the urethane bond formed in each end.

Branched PEG has a polyoxyethylene structure in a portion branched from the main chain having a hydroxyl group at each end. An example of branched PEG is a polyol with a structure in which a hydrogen atom in an alkylene group having a hydroxyl group at each end is replaced with a group having a polyoxyethylene structure. Using branched PEG can introduce a polyoxyethylene structure into side chains rather than the main polymer chain of the polyurethane resin elastomer. Introducing a hydrophilic polyoxyethylene structure into side chains instead of the main chain allows the polyoxyethylene structure to exert its effect more effectively even in only a small amount introduced, thereby increasing the water content and leading to better biocompatibility.

The content of polyethylene glycol is preferably 1 mass% or higher and lower than 50 mass% based on 100 mass% of the constituents of the polyurethane resin elastomer. An overly high content of polyethylene glycol decreases physical properties such as tensile strength, and decreases the strength retention rate when the polyurethane resin elastomer contains water. Thus, the content of polyethylene glycol based on 100 mass% of the constituents is preferably lower than 50 mass%, more preferably 40 mass% or lower, and still more preferably 30 mass% or lower. From the viewpoint of biocompatibility, the content of polyethylene glycol based on 100 mass% of the constituents is preferably 1 mass% or higher, more preferably 3 mass% or higher, and still more preferably 5 mass% or higher or 10 mass% or higher. The content of polyethylene glycol based on 100 mass% of a polyol is not particularly limited, and may be, for example, 5 mass% or higher, 5 to 60 mass%, 8 to 50 mass%, or 10 to 40 mass%.

The polyurethane resin elastomer may contain one or more other polyols as constituents in addition to the polyethylene glycol. The other polyols can be any polyols commonly used in synthesizing polyurethane. Examples include polycarbonate polyol, polyester polyol, polyether polyol, hydrocarbon polyol, and low-molecular-weight polyols with a molecular weight of less than 200. These may be used singly or in a combination of two or more.

The polycarbonate polyol is, for example, a polyol obtained by reacting an aliphatic or alicyclic polyol with a carbonate derivative such as carbonic ester or phosgene. The polycarbonate polyol for use is preferably a polycarbonate diol, and more preferably a polycarbonate diol containing an aliphatic diol as a constituent. Specific examples of aliphatic diols include C₁₋₈ aliphatic diols, such as ethylene glycol, diethylene glycol, 1,4-butanediol, 1,3-butanediol, 2,3-butanediol, 1,3-propylene glycol, 1,2-propylene glycol, 1,6-hexanediol, 3-methyl-1,5-pentanediol, and neopentyl glycol. These may be used singly or in a combination of two or more.

The polyester polyol is, for example, an esterified condensate obtained by reacting a low-molecular-weight polyol (e.g., the aliphatic diols mentioned above) with a polyvalent carboxylic acid. Examples of polyvalent carboxylic acids include succinic acid, glutaric acid, adipic acid, sebacic acid, phthalic acid, isophthalic acid, terephthalic acid, trimellitic acid, and tetrahydrofuroic acid. These may also be used in a combination of two or more.

The polyether polyol is, for example, a polyol obtained by reacting a low-molecular-weight polyol such as polytetramethylene ether glycol (PTMG) or the aliphatic diols mentioned above with alkyne oxide such as ethylene oxide, propylene oxide, or butylene oxide (excluding, however, polyethylene glycol). These may also be used in a combination of two or more.

The hydrocarbon polyol is terminated with hydroxyl groups at the ends of the hydrocarbon chain. Examples include polybutadiene polyol, polyisoprene polyol, hydrogenated polybutadiene polyol, and hydrogenated polyisoprene polyol. These may also be used in a combination of two or more.

Examples of low-molecular-weight polyols with a molecular weight of less than 200 include ethylene glycol, diethylene glycol, triethylene glycol, 1,2-propylene glycol, 1,3-propylene glycol, neopentyl glycol, 1,3-butanediol, 1,4-butanediol, 3-methylpentanediol, 1,6-hexanediol, 1,8-octanediol, 2-methyl-1,3-propanediol, cyclohexane dimethanol, glycerin, and trimethylolpropane. These may also be used in a combination of two or more.

The other polyols to be used with polyethylene glycol are preferably polycarbonate diols because of their generally excellent strength. That is, the polyurethane resin elastomer in a preferable embodiment further contains a polycarbonate diol as a constituent. The content of polycarbonate polyol is not particularly limited, and is preferably 80 parts by mass or more, more preferably 90 parts by mass or more, and still more preferably 95 parts by mass or more, per 100 parts by mass of the polyol component excluding polyethylene glycol. The content of polycarbonate diol based on 100 mass% of the constituents of the polyurethane resin elastomer is not particularly limited, but is preferably 30 to 90 mass%, and more preferably 35 to 80 mass%, or 40 to 75 mass%.

The polyisocyanate is not particularly limited, and various polyisocyanate compounds having two or more isocyanate groups per molecule are usable. Examples of polyisocyanates include aliphatic polyisocyanates, alicyclic polyisocyanates, aromatic polyisocyanates, and modified forms of these polyisocyanates. These can be used singly or in a combination of two or more.

Examples of aliphatic polyisocyanates include tetramethylene diisocyanate, dodecamethylene diisocyanate, hexamethylene diisocyanate (HDI), 2,2,4-trimethyl hexamethylene diisocyanate, 2,4,4-trimethyl hexamethylene diisocyanate, lysine diisocyanate, 2-methylpentane-1,5-diisocyanate, and 3-methylpentane-1,5-diisocyanate. These may also be used in a combination of two or more.

Examples of alicyclic polyisocyanates include isophorone diisocyanate (IPDI), hydrogenated xylylene diisocyanate, methylenebis(4,1-cyclohexylene)=diisocyanate (hydrogenated MDI), 1,4-cyclohexane diisocyanate, methyl cyclohexylene diisocyanate, and 1,3-bis(isocyanatemethyl) cyclohexane. These may also be used in a combination of two or more.

Examples of aromatic polyisocyanates include tolylene diisocyanate (TDI, such as 2,4-TDI, and 2,6-TDI), diphenylmethane diisocyanate (MDI, such as 2,2'-MDI, 2,4'-MDI, and 4,4'-MDI), polymeric MDI, 4,4'-dibenzyl diisocyanate, 1,5-naphthylene diisocyanate, xylylene diisocyanate (XDI), 1,3-phenylene diisocyanate, and 1,4-phenylene diisocyanate. These may also be used in a combination of two or more.

Examples of modified forms of these polyisocyanates include modified isocyanurate, modified allophanate, modified burette, modified adducts, and modified carbodiimide.

In an embodiment, a preferred polyisocyanate may contain at least one member selected from the group consisting of hexamethylene diisocyanate, diphenylmethane diisocyanate, methylenebis(4,1-cyclohexylene)=diisocyanate, isophorone diisocyanate, and modified forms of these. More preferably, the polyisocyanate may contain at least one member selected from the group consisting of hexamethylene diisocyanate, diphenylmethane diisocyanate, methylenebis(4,1-cyclohexylene)=diisocyanate, and modified forms of these.

Although the content of polyisocyanate is not particularly limited, the ratio of isocyanate groups to hydroxyl groups present in the constituents (NCO/OH index, the molar ratio of isocyanate groups to hydroxyl groups) is preferably 0.7 to 1.6, more preferably 0.8 to 1.4, and still more preferably 1.0 to 1.2. The content of polyisocyanate based on 100 mass% of the constituents of the polyurethane resin elastomer is not particularly limited, and may be, for example, 5 to 50 mass%, 8 to 40 mass%, or 10 to 30 mass%.

The polyurethane resin elastomer may further contain a monohydric alcohol containing a fluorine atom ("fluorinated alcohol" below) as a constituent. A fluorinated alcohol can be used to decrease the water contact angle of the polyurethane resin elastomer.

Specific examples of fluorinated alcohols include trifluoroethanol, tetrafluoropropanol, pentafluoropropanol, trifluoromethylpropanol, hexafluorobutanol, heptafluorobutanol, pentafluoropentanol, octafluoropentanol, nonafluoropentanol, tridecafluoroheptanol, nonafluorohexanol, tridecafluorooctanol, heptadecafluorodecanol, tetrafluorobenzyl alcohol, trifluoromethylbenzyl alcohol, trifluoromethoxybenzyl alcohol, and alkylene oxide adducts of these (e.g., ethylene oxide adducts). These may be used singly or in a combination of two or more.

The content of the fluorinated alcohol based on 100 mass% of the constituents of the polyurethane resin elastomer is not particularly limited, and may be, for example, 0.1 to 15 mass%, 1 to 10 mass%, or 3 to 8 mass%.

Various additives, such as catalysts, defoamers, antioxidants, colorants, and UV absorbers, may be added to the polyurethane resin elastomer to the extent that the object of the present embodiment is not compromised. Examples of catalysts for use include various urethane polymerization catalysts, such as metal catalysts (e.g., tin-based catalysts, lead-based catalysts, and bismuth-based catalysts) and amine catalysts.

The polyurethane resin elastomer is preferably thermoplastic. A thermoplastic polyurethane resin elastomer can be plasticized by heating and molded. To obtain a thermoplastic polyurethane resin elastomer, it is preferable to mainly use a bifunctional polyol and a bifunctional polyisocyanate for the constituents. However, as long as the resulting polyurethane resin elastomer is thermoplastic, the polyurethane resin elastomer may contain a tri- or higher functional polyol and/or a tri- or higher functional polyisocyanate.

The polyurethane resin elastomer is preferably an elastomer formed without becoming an emulsion. To emulsify and disperse polyurethane in water to obtain an aqueous dispersion (aqueous emulsion), a surfactant is used as an emulsifier; thus, the resulting polyurethane generally contains a surfactant. However, the presence of a surfactant is a factor that reduces water resistance. From the viewpoint of water resistance, the polyurethane resin elastomer is preferably formed without becoming an emulsion and is surfactant-free.

The water content performance of the polyurethane resin elastomer is not particularly limited. In an embodiment, the maximum water content of the polyurethane resin elastomer at 4°C may be higher than 1 mass% and lower than 55 mass%, or 5 mass% or higher and 50 mass% or lower. In an embodiment, the maximum water content of the polyurethane resin elastomer at 37°C may be higher than 1 mass% and lower than 50 mass%, 3 mass% or higher and 40 mass% or lower, and 5 mass% or higher and 30 mass% or lower. As used herein, the "maximum water content" refers to the ratio of the maximum mass of water that can be absorbed when the polyurethane resin elastomer is immersed in water at 4°C or 37°C to 100 mass% of the polyurethane resin elastomer on a dry basis (saturated water content), and is measured according to the method described in the Examples below.

The hardness of the polyurethane resin elastomer is not particularly limited. In an embodiment, the hardness (shore A) (JIS K7312:1996) of the polyurethane resin elastomer on a dry basis may be 30 or higher and 90 or lower, or 40 or higher and 70 or lower.

The tensile strength (JIS K6251:2017) of the polyurethane resin elastomer is not particularly limited. In an embodiment, the tensile strength of the polyurethane resin elastomer on a dry basis may be 5 MPa or higher and 30 MPa or lower, or 10 MPa or higher and 25 MPa or lower.

The elongation at break (JIS K6251:2017) of the polyurethane resin elastomer is not particularly limited. In an embodiment, the elongation at break of the polyurethane resin elastomer on a dry basis may be 300% or higher and 2000% or lower, or 500% or higher and 1500% or lower.

The glass transition temperature (Tg) of the polyurethane resin elastomer is not particularly limited. In an embodiment, the glass transition temperature of the polyurethane resin elastomer (measured with a differential scanning calorimeter) may be -55°C or higher and -25°C or lower.

The water contact angle of the polyurethane resin elastomer is not particularly limited. In an embodiment, the water contact angle of the polyurethane resin elastomer in a water-containing state may be 20° or greater and 95° or lower, or 30° or greater and 80° or lower. A smaller water contact angle is thought to lead to higher antithrombogenicity.

The method for synthesizing the polyurethane resin elastomer is not particularly limited. The polyurethane resin elastomer can be synthesized in the same manner as for ordinary polyurethanes. Preferably, the polyurethane resin elastomer can be synthesized by reacting polyethylene glycol, another polyol as an optional constituent, and an isocyanate without using a solvent.

The method for molding the polyurethane resin elastomer into the medical device in the present embodiment is not particularly limited. For example, molding may be performed according to a two-component curing method or a thermal melting method.

The two-component curing method is a method of molding a polyurethane resin elastomer into a medical device of a predetermined shape directly from a mixture of liquid A and liquid B when synthesizing the polyurethane resin elastomer by mixing liquid A and liquid B containing the constituents described above. Specifically, in this case, the polyurethane resin elastomer is molded while a polyol is reacted with a polyisocyanate.

For example, the two-component curing method may be performed by mixing liquid A containing polyethylene glycol and another polyol with liquid B containing a polyisocyanate, and curing the mixture while molding it into a predetermined shape according to a known molding method, such as coating or injection molding. For another example, polyethylene glycol is mixed and reacted with a polyisocyanate to obtain an isocyanate-terminated urethane prepolymer, and then the urethane prepolymer (used as liquid B) is mixed with liquid A containing another polyol, followed by curing the mixture while molding it into a predetermined shape according to coating or injection molding. For another example, another polyol and a polyisocyanate are mixed and reacted to obtain an isocyanate-terminated urethane prepolymer, and then the urethane prepolymer (used as liquid B) is mixed with liquid A containing polyethylene glycol, followed by curing the mixture while molding it into a predetermined shape according to coating or injection molding.

The thermal melting method is a method of mixing the constituents to synthesize a polyurethane resin elastomer and then thermally melting the polyurethane resin elastomer by using its thermoplasticity to mold it into a predetermined shape for the medical device.

For example, the thermal melting method may be performed by mixing and curing polyethylene glycol, another polyol, and a polyisocyanate to obtain a cured polyurethane and then thermally melting the cured polyurethane, and further molding the melted polyurethane into a predetermined shape according to a known molding method, such as coating or injection molding. For another example, polyethylene glycol and a polyisocyanate are mixed and reacted to obtain an isocyanate-terminated urethane prepolymer, and then the urethane prepolymer is mixed with another polyol and cured to obtain a cured polyurethane. The cured polyurethane is thermally melted, and the melted polyurethane is molded into a predetermined shape according to a known molding method, such as coating or injection molding. For another example, another polyol and a polyisocyanate are mixed and reacted to obtain an isocyanate-terminated urethane prepolymer, and then the urethane prepolymer is mixed with polyethylene glycol and cured to obtain a cured polyurethane. The cured polyurethane is then thermally melted, and the melted polyurethane is molded into a predetermined shape according to a known molding method, such as coating or injection molding.

The polyurethane resin elastomer may be of any shape as a medical device. The polyurethane resin elastomer may be of a shape that conforms to the entirety of any of the various medical devices described above, or that conforms to at least part or the entirety of the portion that comes into contact with biological tissue or body fluid in those medical devices, or of a film shape that covers at least part or the entirety of such a portion. For example, the polyurethane resin elastomer may be formed into a plate, a column, a hollow cylinder, or even the entirety of a three-dimensional shape such as artificial bone.

In a preferred embodiment, if the polyurethane resin elastomer is formed without becoming an emulsion, the polyurethane resin elastomer can be molded into a coating film or molded article with a large thickness. Thus, the polyurethane resin elastomer is preferably a coating film or molded article with a thickness of more than 1 mm. The coating film or molded article preferably has a thickness of 2 mm or more. The upper limit of the thickness of the coating film or molded article is not particularly limited, and may be 100 mm or less, for example. The thickness of the coating film refers to the film thickness. The "thickness" of a coating film as used herein refers to film thickness. The "thickness" of a molded article refers to the thickness of a plate if the molded article has a plate shape. The "thickness" of a molded article refers to the width between the inner circumference and the outer circumference (wall thickness) if the molded article has a hollow shape. The "thickness" refers to the smallest dimension among length, width, and height if the molded article has a three-dimensional shape, such as a column or artificial bone.

After the polyurethane resin elastomer is molded in the manner above, the polyurethane resin elastomer can be impregnated with water to allow the polyurethane resin elastomer to contain water.

In the medical device in the present embodiment, the water contained in the polyurethane resin elastomer may be pure water, such as distilled water or deionized water, or may be an aqueous solution, such as physiological saline.

The content of water in the polyurethane resin elastomer in a medical device is not particularly limited, and is preferably higher than 1 mass% and lower than 50 mass% based on the polyurethane resin elastomer. As used herein, the content of water is the ratio by mass of water contained in the polyurethane resin elastomer to 100 mass% of the polyurethane resin elastomer on a dry basis. The content of water is preferably 3 to 40 mass%, more preferably 5 to 30%, and still more preferably 10 to 25 mass%.

### Examples

The starting materials used in Examples and Comparative Examples are listed below.

### Polyethylene Glycol

· PEG#200: Manufactured by NOF Corporation, PEG#200 (number average molecular weight: 200)
· PEG-600S: Manufactured by DKS Co., Ltd., PEG-600S (number average molecular weight: 600)
· Ymer N180: Manufactured by Perstorp, Ymer N180 (branched PEG, number average molecular weight: 623)
· PEG#1000: Manufactured by NOF Corporation PEG#1000 (number average molecular weight: 1000)
· Ymer N120: Manufactured by Perstorp, Ymer N120 (branched PEG, number average molecular weight: 1000)
· PEG#2000: Manufactured by NOF Corporation, PEG#2000 (number average molecular weight: 2000)
· PEG#4000: Manufactured by NOF Corporation, PEG#4000 (number average molecular weight: 4000)

Polycarbonate Diol
   · UH-100: Polycarbonate diol of 1,6-hexanediol, manufactured by UBE Corporation, ETERNACOLL UH-100 (number average molecular weight: 1000)
Fluorinated Alcohol
   · Fluorinated alcohol: F(CF₂)₆(CH₂)₂(OCH₂CH₂)₇OH, manufactured by DKS Co., Ltd. (molecular weight: 672)
Dimethylolpropionic Acid
   · Bis-MPA: manufactured by Perstorp, BiS-MPA (molecular weight: 134)
Polyisocyanate
   · HDI: Hexamethylene diisocyanate, manufactured by Asahi Kasei Corporation, Duranate 50M
   · HDI Prepolymer: Bifunctional HDI prepolymer, manufactured by Asahi Kasei Corporation, Duranate D201
   · Modified MDI: Carbodiimide-modified MDI, manufactured by BASF INOAC Polyurethanes, Lupranat MM 103
   · Hydrogenated MDI: Manufactured by Evonik, VESTANAT H12MDI
   · IPDI: Isophorone diisocyanate, manufactured by Evonik, VESTANAT IPDI
Additives
   - Tin-based Catalyst: Manufactured by Nitto Kasei Co., Ltd., NEOSTANN U-810
   - Bismuth-based Catalyst: Manufactured by Nitto Kasei Co., Ltd., NEOSTANN U-600
   - Defoamer: Silicone-based defoamer, manufactured by Shin-Etsu Chemical Co., Ltd., Silicon KS-69
   - Triethyl Amine: neutralizer, manufactured by Daicel Corporation, Triethyl Amine

### Example 2-1

20 parts by mass of PEG-600S and 17 parts by mass of HDI were mixed and stirred at 70°C for 1 hour, thereby obtaining an isocyanate-terminated urethane prepolymer (liquid B). Liquid B had a viscosity of 600 mPa·s (25°C). For liquid A, 63 parts by mass of UH-100, 0.01 parts by mass of a tin-based catalyst, and 0.1 parts by mass of a defoamer were mixed. Liquid B, adjusted to 40°C, and liquid A, adjusted to 70°C, were mixed and placed in a Teflon^{®}-coated petri dish so as to give a film thickness of 2 mm and then cured with heating at 80°C for 16 hours, thereby obtaining a coating film of a polyurethane resin elastomer with a thickness of 2 mm.

### Example 2-2

20 parts by mass of PEG-600S, 63 parts by mass of UH-100, 17 parts by mass of HDI, 0.01 parts by mass of a tin-based catalyst, and 0.1 parts by mass of a defoamer were mixed and cured with heating at 80°C for 16 hours, thereby obtaining a block-shaped cured product with a film thickness of 5 cm. The block-shaped cured product was melted with heating at 190°C and placed in a Teflon^{®}-coated petri dish to give a film thickness of 2 mm, followed by curing it with heating at 80°C for 16 hours, thereby obtaining a coating film of a polyurethane resin elastomer with a thickness of 2 mm.

### Examples 1 and 3 to 14 and Comparative Examples 1 to 4

A coating film of a polyurethane resin elastomer with a thickness of 2 mm of each of Examples 1 and 3 to 14 and Comparative Examples 1 to 4 was obtained in the same manner as in Example 2-1, except that the amount of each constituent was changed as shown in Tables 1 to 3 (parts by mass).

The content of PEG shown in Tables 1 to 3 is the content of polyethylene glycol (mass%) based on 100 mass% of the constituents of the polyurethane resin elastomer. The NCO/OH index is the molar ratio of isocyanate groups to hydroxyl groups contained in the constituents of the polyurethane resin elastomer.

2 mm-thick sheets, which were the coating films of the Examples and Comparative Examples peeled from petri dishes, were measured for physical properties such as hardness, tensile strength, elongation at break, and Tg on a dry basis. The sheets containing water were also measured and evaluated for physical properties such as maximum water content (4°C), maximum water content (37°C), tensile strength retention rate, water contact angle, and intermediate water. The measurement and evaluation methods for these physical properties are described below.

### Physical Properties of Dry Sheet

### Hardness

The hardness (shore A) of each sheet was measured according to JIS K 7312:1996 with a rubber hardness tester (trade name: ASKER Durometer Type A, manufactured by Kobunshi Keiki Co., Ltd.).

### Tensile Strength and Elongation at Break

A tensile test was performed at a temperature of 23°C (relative humidity: 55%) at a tension rate of 300 mm/min on a dumbbell specimen No. 3 cut out of each sheet according to JIS K6251:2017 to measure tensile strength (MPa) and elongation at break (%).

### Glass Transition Temperature Tg

The sheets were measured with a differential scanning calorimeter (Thermo plus EVO DSC8230L, manufactured by Rigaku Corporation) (temperature increase rate: 5°C/min).

### Physical Properties of Sheets Containing Water

### Maximum Water Content: 4°C, and Maximum Water Content: 37°C

The sheets were cut to 2 cm × 4 cm pieces and used as evaluation samples. The mass of each evaluation sample was measured before and after immersion in tap water (test solution, 4°C and 37°C) for 24 hours, and the rate of increase in mass was calculated according to the following formula to determine the rate of increase in mass to be the maximum water content (4°C) and maximum water content (37°C) of a polyurethane resin elastomer. Rate of increase in mass (%) = {(mass after immersion - mass before immersion)/mass before immersion] × 100

### Tensile Strength Retention Rate

Each sheet was maintained at room temperature (25°C) for 7 days while being immersed in water. Immediately after the sheet was removed from the water, the tensile strength was measured in the same manner as for tensile strength of a sheet on a dry basis described above. The tensile strength retention rate was determined according to the following formula and evaluated according to the following evaluation criteria. Tensile strength retention rate (%) = (tensile strength after immersion in water/tensile strength before immersion in water) × 100

### · Evaluation Criteria

A: Tensile strength retention rate of 60% or higher
B: Tensile strength retention rate of 40% or higher and lower than 60
C: Tensile strength retention rate of lower than 40

### Water Contact Angle

Each sheet was maintained at room temperature (25°C) for 7 days while being immersed in water. Immediately after the sheet was removed from the water, the water contact angle was measured. Water was applied to the surface of the sheet immediately after it was removed from the water, and the contact angle (°) was measured with a contact angle meter (Drop Master 500, contact angle meter manufactured by Kyowa Interface Science Co., Ltd.). This contact angle is referred to as "water contact angle."

### Intermediate Water

Differential scanning calories were measured with a differential scanning calorimeter (Thermo plus EVO DSC8230L, manufactured by Rigaku Corporation) to evaluate whether an endothermic peak derived from intermediate water was present during the temperature increase process. The presence of an endothermic peak is indicated by "A," which means the presence of intermediate water, whereas the absence of an endothermic peak is indicated by "C," which means the absence of intermediate water.

The procedure and conditions for measuring differential scanning calories are described below.

### · Measurement Procedure

(1) The sheets were each immersed in pure water at 4°C for more than 72 hours to prepare samples containing water.
(2) Each sample containing water was taken out and immediately measured.
(3) About 5 mg of the sample was collected in an aluminum pan for DSC testing, and differential scanning calories were measured in a nitrogen atmosphere.
   - DSC Measurement Conditions

The measurement temperature ranged from -100°C to 37°C. The temperature program was set as follows: The temperature descends from 37°C to -100°C at 5°C/minutes, and is maintained at -100°C for 5 minutes, after which the temperature increases from -100°C to 37°C at 5°C/minutes.

**Table 1**

| | Examples | | | | | | | |
|---|---|---|---|---|---|---|---|---|
| | 1 | 2-1 | 2-2 | 3 | 4 | 5 | 6 | 7 |
| Formula (parts by mass) | | | | | | | | |
| PEG#200 | 10 | | | | | | | |
| PEG-600S | | 20 | 20 | 20 | | | | |
| Ymer N180 | | | | | 20 | | | |
| PEG#1000 | 10 | | | | | 20 | | 10 |
| Ymer N120 | | | | | | | 20 | |
| PEG#2000 | | | | | | | | 10 |
| PEG#4000 | | | | | | | | |
| UH-100 | 63 | 63 | 63 | 63 | 63 | 65 | 65 | 66 |
| Fluorinated Alcohol | | | | | | | | |
| Bis-MPA | | | | | | | | |
| HDI | 17 | 17 | 17 | 17 | 17 | 15 | 15 | 14 |
| HDI Prepolymer | | | | | | | | |
| Modified MDI | | | | | | | | |
| Hydrogenated MDI | | | | | | | | |
| IPDI | | | | | | | | |
| Tin-based Catalyst | 0.01 | 0.01 | 0.01 | | 0.01 | 0.01 | 0.01 | 0.01 |
| Bismuth-based Catalyst | | | | 0.01 | | | | |
| Triethylamine | | | | | | | | |
| Defoamer | 0.1 | 0.1 | 0.1 | 0.1 | 0.1 | 0.1 | 0.1 | 0.1 |
| Total | 100.11 | 100.11 | 100.11 | 100.11 | 100.11 | 100.11 | 100.11 | 100.11 |
| PEG Content (mass%) | 20 | 20 | 20 | 20 | 20 | 20 | 20 | 20 |
| NCO/OH Index | 1.05 | 1.05 | 1.05 | 1.07 | 1.07 | 1.05 | 1.05 | 1.03 |

| Physical Properties of Dry Sheet | | | | | | | | |
|---|---|---|---|---|---|---|---|---|
| Hardness | 50 | 50 | 53 | 55 | 50 | 55 | 45 | 65 |
| Tensile Strength (MPa) | 18 | 20 | 21 | 19 | 16 | 21 | 22 | 12 |
| Elongation at Break (%) | 800 | 900 | 850 | 900 | 1000 | 1200 | 1400 | 600 |
| Tg (°C) | -40 | -40 | -40 | -40 | -43 | -46 | -48 | -50 |

| Physical Properties of Sheets Containing Water | | | | | | | | |
|---|---|---|---|---|---|---|---|---|
| Maximum Water Content (4°C) (%) | 25 | 20 | 21 | 22 | 27 | 35 | 42 | 46 |
| Maximum Water Content (37°C) (%) | 15 | 15 | 16 | 13 | 20 | 26 | 28 | 31 |
| Tensile Strength Retention Rate (%) | A | A | A | A | A | B | B | B |
| Water Contact Angle (°) | 70 | 70 | 69 | 69 | 69 | 65 | 63 | 64 |
| Intermediate Water | A | A | A | A | A | A | A | A |

**Table 2**

| | Examples | | | | | | |
|---|---|---|---|---|---|---|---|
| | 8 | 9 | 10 | 11 | 12 | 13 | 14 |
| Formula (parts by mass) | | | | | | | |
| PEG#200 | | | | | | | |
| PEG-600S | 20 | 20 | 20 | 20 | 40 | 5 | 20 |
| Ymer N180 | | | | | | | |
| PEG#1000 | | | | | | | |
| Ymer N120 | | | | | | | |
| PEG#2000 | | | | | | | |
| PEG#4000 | | | | | | | |
| UH-100 | 39 | 54 | 55 | 59 | 41 | 80 | 58 |
| Fluorinated Alcohol | | | | | | | 5 |
| Bis-MPA | | | | | | | |
| HDI | | | | | 19 | 15 | 17 |
| HDI Prepolymer | 41 | | | | | | |
| Modified MDI | | 26 | | | | | |
| Hydrogenated MDI | | | 25 | | | | |
| IPDI | | | | 21 | | | |
| Tin-based Catalyst | 0.01 | 0.01 | 0.01 | 0.01 | 0.01 | 0.01 | 0.01 |
| Bismuth-based Catalyst | | | | | | | |
| Triethylamine | | | | | | | |
| Defoamer | 0.1 | 0.1 | 0.1 | 0.1 | 0.1 | 0.1 | 0.1 |
| Total | 100.11 | 100.11 | 100.11 | 100.11 | 100.11 | 100.11 | 100.11 |
| PEG Content (mass%) | 20 | 20 | 20 | 20 | 40 | 5 | 20 |
| NCO/OH Index | 1.07 | 1.05 | 1.08 | 1.02 | 1.05 | 1.01 | 1.07 |

| Physical Properties of Dry Sheet | | | | | | | |
|---|---|---|---|---|---|---|---|
| Hardness | 55 | 70 | 65 | 52 | 40 | 65 | 60 |
| Tensile Strength (MPa) | 13 | 24 | 20 | 8 | 12 | 24 | 22 |
| Elongation at Break (%) | 950 | 600 | 650 | 400 | 700 | 1000 | 1100 |
| Tg (°C) | -35 | -31 | -35 | -32 | -35 | -42 | -36 |

| Physical Properties of Sheets Containing Water | | | | | | | |
|---|---|---|---|---|---|---|---|
| Maximum Water Content (4°C) (%) | 21 | 18 | 20 | 22 | 44 | 7 | 23 |
| Maximum Water Content (37°C) (%) | 13 | 11 | 13 | 14 | 29 | 5 | 17 |
| Tensile Strength Retention Rate (%) | A | A | A | A | B | A | A |
| Water Contact Angle (°) | 66 | 74 | 74 | 91 | 63 | 85 | 31 |
| Intermediate Water | A | A | A | A | A | A | A |

**Table 3**

| | Comparative Examples | | | | |
|---|---|---|---|---|---|
| | 1 | 2 | 3 | 4 | 5 |
| Formula (parts by mass) | | | | | |
| PEG#200 | | | | | |
| PEG-600S | | | 60 | 77 | |
| Ymer N180 | | | | | |
| PEG#1000 | | | | | |
| Ymer N120 | | | | | |
| PEG#2000 | | | | | |
| PEG#4000 | | | | | 95.7 |
| UH-100 | 85 | 59 | 19 | | |
| Fluorinated Alcohol | | | | | |
| Bis-MPA | | 11 | | | |
| HDI | 15 | 25 | 21 | 23 | 4.3 |
| HDI Prepolymer | | | | | |
| Modified MDI | | | | | |
| Hydrogenated MDI | | | | | |
| IPDI | | | | | |
| Tin-based Catalyst | 0.01 | 0.01 | 0.01 | 0.01 | 0.01 |
| Bismuth-based Catalyst | | | | | |
| Triethylamine | | 5 | | | |
| Defoamer | 0.1 | 0.1 | 0.1 | 0.1 | 0.1 |
| Total | 100.11 | 100.11 | 100.11 | 100.11 | 100.11 |
| PEG Content (mass%) | 0 | 0 | 60 | 77 | 96 |
| NCO/OH Index | 1.05 | 1.06 | 1.05 | 1.06 | 1.07 |

| Physical Properties of Dry Sheet | | | | | |
|---|---|---|---|---|---|
| Hardness | 65 | 75 | 60 | 54 | 90 |
| Tensile Strength (MPa) | 24 | 10 | 4 | 4 | 0.2 |
| Elongation at Break (%) | 900 | 350 | 400 | 400 | 10 |
| Tg (°C) | -43 | -26 | -32 | -30 | -65 |

| Physical Properties of Sheets Containing Water | | | | | |
|---|---|---|---|---|---|
| Maximum Water Content (4°C) (%) | 1 | 1 | 60 | 110 | Not Measurable |
| Maximum Water Content (37°C) (%) | 1 | 1 | 38 | 60 | Not Measurable |
| Tensile Strength Retention Rate (%) | A | B | C | C | Not Measurable |
| Water Contact Angle (°) | 98 | 85 | 66 | 63 | Not Measurable |
| Intermediate Water | C | C | A | A | Not Measurable |

The results are as shown in Table 1. In Comparative Examples 1 and 2, the water content was small, and no intermediate water was present due to no polyethylene glycol contained in the constituents of the polyurethane resin elastomer. In Comparative Examples 3 and 4, an overly high amount of polyethylene glycol resulted in low tensile strength, and also a low strength retention rate of the coating film containing water. In Comparative Example 5, an overly large molecular weight of polyethylene glycol resulted in small tensile strength and made the water-containing coating film gelatinous; thus, the physical properties of the coating film containing water could not be measured.

In contrast, Examples 1 to 14 showed the presence of intermediate water while demonstrating high levels of tensile strength and elongation at break, and also an excellent strength retention rate of the coating films containing water.

### Platelet Adhesion Test

A platelet adhesion test was performed on each sheet produced in Example 2-1, Example 8, Example 14, Comparative Example 1, and Comparative Example 2. The platelet adhesion test was performed according to the following method.

Human whole blood, purchased experimental blood collected in the United States, was used for the experiment within 5 days after collection. The human whole blood that had been refrigerated was allowed to stand at room temperature for about 30 minutes to be brought to room temperature. The blood was then inverted for blending three times and centrifuged at 1500 rpm for 5 minutes with a centrifugal separator (tabletop centrifuge model 2420, Kubota Corporation). About 500 µL of the (translucent pale yellow) supernatant was collected and used as a platelet-rich plasma (PRP). After collection, centrifugation was further performed at 4000 rpm for 10 minutes, and about 2 mL of the (transparent pale yellow) supernatant was collected and used as a platelet-poor plasma (PPP). The number of platelets in PRP diluted 800-fold with PBS (-) was counted using a hemocytometer to calculate the platelet concentration in PRP. The PRP was diluted with PPP to a seeding concentration of 3.0 × 10⁷ cells/cm² to prepare a platelet suspension.

The sheets prepared in the Examples and Comparative Examples were each thoroughly moistened beforehand at room temperature (25°C) with physiological saline. 450 µL (about 300 µL/cm²) of the prepared platelet suspension was placed on the surface of each sheet and incubated at 37°C for 1 hour to allow platelets to adhere to the sheet. Thereafter, the platelet suspension was removed, and the sheet was washed with PBS twice; then, the sheet was immersed in a 1% glutaraldehyde solution (25% glutaraldehyde, Polysciences, Inc., 01909 diluted to 1/25 with PBS (-)) and incubated at 37°C for 2 hours to immobilize the adhered platelets onto the sheet. After immobilization, the sheet was washed by immersing it in PBS (-) (10 minutes), a mixture of PBS (-) and water in a ratio of 1:1 (8 minutes), and water (8 minutes and 10 minutes) one time each. After washing, the sheet was air-dried for 3 hours, and then dried in a vessel containing silica gel for at least 1 day. After drying, the surface of the sheet was observed with a scanning electron microscope (SEM, KEYENCE, VE-9800 3D real surface view microscope) to count the number of platelets adhered to the surface of each sheet.

Platelets were allowed to adhere to the surface of a PET (polyethylene terephthalate) film, which is known to not show hemocompatibility, in the same manner as above. The number of platelets adhered to the surface of a PET was counted and used as a negative control. A value standardized by dividing the number of platelets adhered to the surface of a sheet by the counted number of platelets on the PET surface was defined as a "platelet adhesion index." The platelet adhesion index excludes the difference in the frequency of platelet adhesion arising from the condition of blood used for evaluation, thus enabling proper evaluation of the level of hemocompatibility indicated by a sample surface.

To clarify the levels of platelet adhesion to a biocompatible surface, a platelet adhesion test was performed, simultaneously with the test described above, on PMEA (poly-2-methoxyethyl acrylate), which is known to have a high level of biocompatibility, and a polymer obtained by copolymerizing MPC (2-methacryloyloxyethyl phosphorylcholine) with BMA (butyl methacrylate) and making it water-insoluble. These were used as positive controls.

Table 4 shows the results. Table 4 also shows the amount of constituents, the physical properties evaluation, and the results of measuring the maximum water content (25°C) of Example 2-1, Example 8, Example 14, Comparative Example 1, and Comparative Example 2 shown in Tables 1 to 3. The maximum water content (25°C) was measured in the same manner as for the maximum water content (4°C) described above, except that the temperature of the tap water for 24 hr immersion of an evaluation sample was set to 25°C.

**Table 4**

| | Example 2-1 | Example 8 | Example 14 | Comparative Example 1 | Comparative Example 2 | PMEA | MPC | PET |
|---|---|---|---|---|---|---|---|---|
| Formula (parts by mass) | | | | | | | | |
| PEG-600S | 20 | 20 | 20 | | | | | |
| UH-100 | 63 | 39 | 58 | 85 | 63 | | | |
| Fluorinated Alcohol | | | 5 | | | | | |
| Bis-MPA | | | | | 11 | | | |
| HDI | 17 | | 17 | 15 | 26 | | | |
| HDI Prepolymer | | 41 | | | | | | |
| Tin-based Catalyst | 0.01 | 0.01 | 0.01 | 0.01 | | | | |
| Triethylamine | | | | | 5 | | | |
| Defoamer | 0.1 | 0.1 | 0.1 | 0.1 | 0.1 | | | |
| Total | 100.11 | 100.11 | 100.11 | 100.11 | 100.11 | | | |
| PEG Content (mass%) | 20 | 20 | 20 | 0 | 0 | | | |

| Physical Properties of Dry Sheet | | | | | | | | |
|---|---|---|---|---|---|---|---|---|
| Hardness | 50 | 55 | 60 | 65 | 75 | | | |
| Tensile Strength (MPa) | 20 | 13 | 22 | 24 | 10 | | | |
| Elongation at Break (%) | 900 | 950 | 1100 | 900 | 350 | | | |
| Tg (°C) | -40 | -35 | -36 | -43 | -26 | | | |

| Physical Properties of Sheets Containing Water | | | | | | | | |
|---|---|---|---|---|---|---|---|---|
| Maximum Water Content (4°C) (%) | 20 | 21 | 23 | 1 | 1 | | | |
| Maximum Water Content (25°C) (%) | 18 | 18 | 21 | 1 | 1 | | | |
| Maximum Water Content (37°C) (%) | 15 | 13 | 17 | 1 | 1 | | | |
| Tensile Strength Retention Rate (%) | A | A | A | A | B | | | |
| Water Contact Angle (°) | 70 | 66 | 31 | 98 | 85 | | | |
| Intermediate Water | A | A | A | C | C | | | |
| Platelet Adhesion Index | 3.8 | 4.3 | 1.3 | 100 | 105 | 0.45 to 15.38 | 0.83 to 15.27 | 100 |

As shown in Table 4, Comparative Examples 1 and 2, in which the polyurethane resin elastomers did not contain polyethylene glycol as a constituent, demonstrated platelet adhesion equivalent to that of PET, is not biocompatible. In contrast, Examples 2-1, 8, and 14, in which the polyurethane resin elastomers contained polyethylene glycol as a constituent, demonstrated decreased adsorption of platelets, with a platelet adhesion index equivalent to or lower than that of PMEA or MPC polymers having high levels of biocompatibility (about 15 or below). This indicates that the polyurethane resin elastomers of these Examples exhibit high levels of biocompatibility and can achieve both physical properties such as biocompatibility and tensile strength.

Additionally, whereas Comparative Examples 1 and 2, which had a high platelet adhesion index and did not show biocompatibility, had no intermediate water, Examples 2-1, 8, and 14, which had a low platelet adhesion index and showed biocompatibility, demonstrated the presence of intermediate water. This indicates that the polyurethane resin elastomers in other Examples that demonstrated the presence of intermediate water would similarly have a low platelet adhesion index and a high level of biocompatibility, thus achieving both biocompatibility and physical properties.

The numerical ranges described in the specification can be combined with their upper and lower limits in any way, and all such combinations shall be listed in the present specification as preferred numerical ranges. The numerical range "X to Y" means X or greater and Y or less.

Although several embodiments of the present invention are described above, these embodiments are simply presented as examples and are not intended to limit the scope of the invention. These embodiments can be implemented in various other forms, and omission, replacement, and modification can be made in various ways without departing from the spirit of the invention. These embodiments and their omission, replacement, and modification are as much within the scope of the claims and their equivalents as within the scope and spirit of the invention.

## Claims

1. A medical device comprising
a polyurethane resin elastomer, and
water contained in the polyurethane resin elastomer,
the polyurethane resin elastomer comprising
polyethylene glycol, and
a polyisocyanate as constituents,
wherein
the polyethylene glycol has a number average molecular weight of 200 or more and 2200 or less, and
the polyethylene glycol is present in an amount of 1 mass% or higher and lower than 50 mass% based on 100 mass% of the constituents of the polyurethane resin elastomer.

2. The medical device according to claim 1, wherein the content of the water is higher than 1 mass% and lower than 50 mass% based on the polyurethane resin elastomer.

3. The medical device according to claim 1 or 2, wherein the polyisocyanate contains at least one member selected from the group consisting of hexamethylene diisocyanate, diphenylmethane diisocyanate, methylenebis(4,1-cyclohexylene)=diisocyanate, isophorone diisocyanate, and modified forms thereof.

4. The medical device according to any one of claims 1 to 3, wherein the polyurethane resin elastomer further comprises a polycarbonate diol as a constituent.

5. The medical device according to any one of claims 1 to 4, wherein the polyurethane resin elastomer is a coating film or molded article with a thickness of more than 1 mm.

6. The medical device according to any one of claim 1 to 5, wherein the polyurethane resin elastomer is formed without becoming an emulsion.

7. The medical device according to any one of claims 1 to 6, wherein the polyurethane resin elastomer is obtained by performing molding while reacting a polyol with the polyisocyanate.

8. The medical device according to any one of claims 1 to 7, wherein the polyurethane resin elastomer is thermoplastic.

9. The medical device according to any one of claims 1 to 8, wherein the polyurethane resin elastomer further comprises a monohydric alcohol containing a fluorine atom as a constituent.
